# EUROPÄISCHE PATENTSCHRIFT

(11) **EP 0 963 446 B1**
(45) Veröffentlichungstag und Bekanntmachung des Hinweises auf die Patenterteilung: **02.05.2003**
(21) Anmeldenummer: 98912372.4
(22) Anmeldetag: 23.02.1998
(51) Int. Cl.: C14C 3/20, C08G 12/36, C14C 3/28

(54) **ZUSAMMENSETZUNG ZUR BEHANDLUNG VON GEGERBTEM LEDER, SOWIE IHRE HERSTELLUNG**
COMPOSITION FOR TREATING TANNED LEATHER, AND ITS PREPARATION
COMPOSITION DE TRAITEMENT DE CUIR TANNE, ET SA PREPARATION

(30) Priorität: 26.02.1997 DE 19707713
(43) Veröffentlichungstag der Anmeldung: 15.12.1999
(73) Patentinhaber: BASF AKTIENGESELLSCHAFT, 67056 Ludwigshafen (DE)
(72) Erfinder: WOLF, Gerhard, D-68775 Ketsch (DE); IGL, Georg, D-71554 Weissach (DE); GRÖSSER, Thomas, D-67067 Ludwigshafen (DE); STÜBINGER, Adolf, D-67227 Frankenthal (DE); WERNER, Jürgen, D-67098 Bad Dürkheim (DE); GUENTHER, Erhard, D-67454 Hassloch (DE)
(74) Vertreter: Isenbruck, Günter, Dr.
(86) Internationale Anmeldenummer: EP9801025
(87) Internationale Veröffentlichungsnummer: WO98038341

(56) Entgegenhaltungen:
- DE-A- 1 545 192
- DE-B- 1 126 888
- FR-A- 2 163 108
- FR-A- 2 270 327

## Beschreibung

Die Erfindung betrifft eine Zusammensetzung enthaltend (A) ein oder mehrere aromatische Sulfonate, (B) ein oder mehrere Aldehydharze und (C) einen Puffer, die Herstellung dieser Zusammensetzung und die Verwendung zur Behandlung, insbesondere zur Neutralisation von mit Mineralstoff- oder Mineralstoff-freien Verbindungen gegerbtem Leder.

Für die Herstellung von Leder und Pelzen werden tierische Häute mit Gerbstoffen gegerbt, wodurch die Häute in widerstandsfähige Leder oder Pelze umgewandelt werden. Die Gerbstoffe, die im wesentlichen eine Vernetzung der Kollagenfasern der Haut bewirken, lassen sich in anorganische bzw. mineralische und organisch-chemische Gerbstoffe einteilen. Die organisch-chemischen Gerbstoffe können zudem synthetischen oder pflanzlichen Ursprungs sein.

Die Mineral- und insbesondere die sogenannte Chromgerbung besitzt für die Herstellung von Leder und Pelzen eine herausragende Stellung. So sind beispielsweise 90% aller Leder chromgegerbt. Bei der Chromgerbung nimmt die Haut Chrom(III)-Salze auf, die die Kollagenfasern aufgrund einer Komplexbildung mit den Carboxy-Gruppen des Kollagens vernetzt. Die hierbei erzeugten Zwischenprodukte der Chromgerbung kommen als Halbfabrikate, sogenannte "Wetblues", in den Handel und sind in idealer Weise geeignet, in die unterschiedlichsten Lederartikel weiterverarbeitet zu werden. Hierzu ist in aller Regel eine sogenannte Nachgerbung notwendig, in der der eigentliche Ledercharakter festgelegt wird.

Die Nachgerbung wird in der Regel mit anionischen Substanzen durchgeführt, die aus der Klasse der synthetischen oder pflanzlichen Gerbstoffe, wie z. B. auch Harzgerbstoffe, Ligninsulfonate, polymere Gerbstoffe, Aldehyde, Paraffinsulfochloride, Polyphosphate oder inerte Füllmittel, stammen können. Zur Herstellung von geschmeidigem und wasserabstoßendem Leder werden in der Nachgerbung auch anionische Fettungsmittel und zur Herstellung von gefärbtem Leder anionische Farbstoffe verwendet.

Ein grundsätzliches Problem bei der Nachgerbung von beispielsweise Chromleder mit anionischen Substanzen ist jedoch, daß der kationische Charakter des gegerbten Leders die anionischen Substanzen sehr schnell abreagieren läßt und dadurch bewirkt, daß die Substanzen nicht oder nicht tief genug in das Leder eindringen können. Dies führt im allgemeinen zu einer oberflächlichen und ungleichmäßigen Ablagerung der Substanzen auf dem Leder und einer Überladung des Narbes. Die Folgen können ein unschönes Narbenbild, Losnarbigkeit, verschlechterte Ledereigenschaften und Narbenbrüchigkeit sein. Sogar eine Ausfällung der Substanzen auf der Lederoberfläche ist möglich.

In der Regel wird daher vor der Nachgerbung von gegerbtem Leder eine Neutralisation oder Entsäuerung vorgeschaltet, die den kationischen Charakter des Leders abschwächen bzw. vermindern soll, aber darüberhinaus auch in den Chemismus der Gerbung eingreifen kann. Die Neutralisation soll daher in ihrer Intensität weder zu knapp noch zu stark sein. Eine ungenügende Neutralisation löst die oben genannten Probleme nur ungenügend und kann beispielsweise auch eine Ursache für eine schlechte Lagerbeständigkeit der Leder sein. Eine Überneutralisation ist hingegen oft die Ursache für eine Losnarbigkeit der Leder.

Eine Überneutralisation wird leicht mit stark wirkenden Neutralisationsmitteln, wie z. B. Soda, Natriumbicarbonat oder Borax erreicht, die daher heutzutage oft in Kombination mit milder wirkenden Mitteln Verwendung finden. Mit mild wirkenden Neutralisationsmitteln, wie z. B. Natrium- und Calciumformiat, Natriumacetat, Natriumsulfit, Natriumthiosulfat oder Natriumpolyphosphat, ist eine Überneutralisation im allgemeinen ausgeschlossen. Nachteil dieser Mittel ist jedoch, daß der bei gewissen Lederarten erwünschte pH-Wert nicht erreicht werden kann.

Weiterhin vorteilhaft bei der Neutralisation ist die Verwendung von Salzen organischer und anorganischer Säuren mit Pufferwirkung, wie z. B. Formiate, Acetate, Oxalate, Adipinate, Glutarate, Citrate, Lactate, Carbonate, Bicarbonate, Polycarbonate, Oxicarbonate, Silikate, Borate oder Phosphate, da der für gewisse Lederarten gewünschte pH-Bereich genauer gesteuert werden kann.

Neben den Salzen mit Pufferwirkung werden auch häufig Gerbsulfonsäuren, insbesondere auf der Basis von Naphthalinsulfonsäure-Formaldehyd-Kondensaten, in Kombination mit Salzen von Säuren mit Pufferwirkung verwendet. Hierbei tritt eine Wechselwirkung der Gerbsulfonsäuren mit beispielsweise den Chromkomplexen unter Verdrängung von Sulfato-Gruppen und Freisetzung von Schwefelsäure ein. Die freigesetzte Schwefelsäure wird durch den Puffer neutralisiert. Nachteil derartiger Mittel ist jedoch, daß die Leder stärker gebleicht werden und daher bei einer gewünschten Färbung der Leder ein Mehrverbrauch an Farbstoff nötig ist. Zudem sind die Färbungen weniger brilliant. Auch werden die Leder in der Regel weicher und weniger festnarbig, was besonders für den Schuhoberledersektor nachteilig ist.

Aufgabe der vorliegenden Erfindung ist daher, eine Zusammensetzung und/oder ein Verfahren zu finden, bei dem die oben beschriebenen Nachteile verhindert oder zumindest verringert werden können.

Ein Gegenstand der Erfindung ist daher eine Zusammensetzung enthaltend (A) ein oder mehrere aromatische Sulfonate; (B) ein oder mehrere Aldehydharze; und (C) einen oder mehrere Puffer.

Hierbei ist zu erwähnen, daß gerade die Kombination der einzelnen Komponenten (A), (B) und (C) und insbesondere die Verwendung von Aldehydharzen als Komponente (B), vor allem die Verwendung von Aldehydharzen nach deren Umsetzung mit Salzen der schwefligen Säure die überraschenden Vorteile der erfindungsgemäßen Zusammensetzung bewirken. Eine weitere vorteilhafte Verbesserung der erfindungsgemäßen Zusammensetzung kann durch einen höheren Sulfonierungsgrad der aromatischen Sulfonate als Komponente (A) erreicht werden.

Das aromatische Sulfonat gemäß (A) ist im allgemeinen ausgewählt aus einem sulfonierten Benzol und seine Derivate, insbesondere Toluol, Phenol, Kresol, Resorcin; sulfonierte polycyclische aromatische Verbindungen und ihre Derivate, insbesondere Naphthalin, Naphthol, (Hydroxy)Diarylsulfon, (Hydroxy)Diarylether, wobei Aryl vorzugsweise Phenyl bedeutet; sulfonierte Oligophenyle oder seine Derivate, vorzugsweise Diphenyl oder Terphenyl; oder Mischungen davon; Insbesondere sind Naphthalin, Toluol und Xylol als Ausgangsstoffe geeignet.

In einer weiteren Ausführungsform ist das aromatische Sulfonat gemäß (A) ein Kondensationsprodukt aus einem oder mehreren aromatischen Sulfonaten und einem aliphatischen Aldehyd, vorzugsweise Formaldehyd und/oder Acetaldehyd, insbesondere Formaldehyd, oder ein Kondensationsprodukt aus einem oder mehreren aromatischen Sulfonaten und einem Kondensationsprodukt aus einem oder mehreren Amiden der Kohlensäure und einem aliphatischen Aldehyd, vorzugsweise Formaldehyd und/oder Acetaldehyd, insbesondere Formaldehyd. Als Amide der Kohlensäure eignen sich vor allem Harnstoff und/oder Derivate des Harnstoffs, wie z. B. Semicarbazid, Guanidin, Dicyandiamid und/oder Dicyandiamid-Derivate, wie z.B. Melamin. Besonders bevorzugt sind ein Harnstoff-Formaldehyd-Kondensationsprodukt, ein Melamin-Formaldehyd-Kondensationsprodukt und/oder ein Melamin-Harnstoff-Formaldehyd-Kondensationsprodukt, vor allem ein Harnstoff-Formaldehyd-Kondensationsprodukt.

Vorteilhafterweise wird die sulfonierte aromatische Verbindung, die einfach oder mehrfach sulfoniert sein kann, aus einem Moläquivalent einer aromatischen Verbindung und 1-5, vorzugsweise 2-4, insbesondere ca. 3-4 Moläquivalente SO₃ hergestellt. Vorzugsweise ist das aromatische Sulfonat eine Mischung aus einfach und mehrfach, insbesondere eine Mischung aus unterschiedlich mehrfach, vor allem zwei- bis dreifach sulfonierten Verbindungen. Als SO₃-Quelle dient vorzugsweise konzentrierte Schwefelsäure, beispielsweise 95-98%ige Schwefelsäure, oder rauchende Schwefelsäure, beispielsweise Oleum 24% (2 mol SO₃). Die Umsetzung mit konzentrierter bzw. rauchender Schwefelsäure erfolgt vorzugsweise bei ca. 100-160 °C, insbesondere bei ca. 130-150 °C ca. 1-4, vorzugsweise ca. 2 Stunden lang. Die Sulfonierungsprodukte sind bei diesem bevorzugten Verfahren im allgemeinen mehrfach sulfoniert, wobei je nach molekularem Verhältnis von aromatischer Verbindung und SO₃ zusätzlich bis zu 50 Gew-% freies Sulfat im Endprodukt enthalten sein kann. Anschließend wird im allgemeinen das Produkt vorzugsweise mit Natronlauge auf einen pH von ca. 6-10 eingestellt.

Die Herstellung von Kondensationsprodukten aus einer sulfonierten aromatischen Verbindung und Formaldehyd bzw. Formaldehyd-Harnstoff-Kondensat sind allgemein bekannt und beispielsweise in DE 28 43 233 am Beispiel eines β-Naphtholsulfonsäure-Formaldehyd- oder eines Naphthalinsulfonsäure-Formaldehyd-Kondensationsproduktes oder in DE 21 13 096 am Beispiel eines Naphthalinsulfonsäure-Formaldehyd-Harnstoff-Kondensationsproduktes näher beschrieben.

In einer anderen bevorzugten Ausführungsform wird das Aldehydharz gemäß (B) durch Kondensation eines oder mehrerer aliphatischer Aldehyde, vorzugsweise Formaldehyd und/oder Acetaldehyd, insbesondere Formaldehyd, mit einer oder mehreren stickstoffhaltigen Verbindungen umgesetzt, vor allem wird das Aldehydharz durch gleichzeitige oder nachfolgende Kondensation eines oder mehrerer aliphatischer Aldehyde mit einer oder mehreren stickstoffhaltigen Verbindungen und einem oder mehreren Salzen der schwefligen Säure, vor allem mit Natriumhydrogensulfit, umgesetzt. Vorteilhafterweise ist die stickstoffhaltige Verbindung Harnstoff, Melamin, Dicyanamid und/oder Guanidin, vorzugsweise Harnstoff. Beispielsweise werden ca. ein Moläquivalent einer stickstoffhaltigen Verbindung, vorzugsweise Harnstoff, mit ca. 2-3 Moläquivalenten eines aliphatischen Aldehyds, vorzugsweise Formaldehyd, bei einem pH-Wert von ca. 7-10 und bei ca. 70-90 °C ca. 1-3 Stunden lang umgesetzt und das Kondensationsprodukt vorzugsweise anschließend mit ca. 1-3 Moläquivalente eines oder mehrerer Salze der schwefligen Säure, vorzugsweise Natriumhydrogensulfit, bei einem pH-Wert von ca. 7-10 und bei ca. 90-105 °C ca. 2-4 Stunden lang umgesetzt. Die Herstellung eines Kondensationsproduktes aus Melamin, Harnstoff, Formaldehyd und Hydrogensulfit ist beispielsweise in EP 0 063 319 näher beschrieben.

In wiederum einer anderen bevorzugten Ausführungsform enthält der oder die Puffer gemäß (C) ein oder mehrere Salze von einer oder mehreren Säuren mit Pufferwirkung, vorzugsweise in einem pH-Bereich von ca. 3-7, insbesondere von ca. 3-5. Die Auswahl des geeigneten Puffers für das entsprechende Leder läßt sich beispielsweise anhand von Titrationskurven leicht bestimmen und ist beispielsweise auch in Bibliothek des Leders, Band 3, Seiten 211-223 (ed. H. Herfeld, Umschau-Verlag/Frankfurt, 1985) näher beschrieben. Insbesondere ist es bevorzugt, wenn die genannte Säure eine organische Säure, vor allem eine organische Mono- und/oder Dicarbonsäure ist. Beispielsweise ist das Salz der organischen Säure vorzugsweise ein Formiat, Acetat, Carbonat, Oxicarbonat, Bicarbonat, Polycarbonat, Citrat, Glutamat, Lactat, Adipinat, Glutarat, Succinat, Oxalat, Malonat, Tartrat, Phthalat, Fumarat, Maleinat, wobei die Natriumsalze besonders bevorzugt sind. Vor allem ist eine Mischung aus Succinat, Glutarat und Adipinat mit einem Gehalt von vorzugsweise ca. 20-30 Gew-% Succinat, ca. 40-50 Gew.-% Glutarat und ca. 20-30 Gew.-% Adipinat und gegebenenfalls zusätzlich ca. 10-80 Gew.-% Formiat bevorzugt. Es ist auch bevorzugt, wenn die genannte Säure eine anorganische Säure, insbesondere schweflige Säure, Phosphorsäure, Kieselsäure, Borsäure und/oder salpetrige Säure ist.

Die Anteile der einzelnen Komponenten (A), (B) und (C) der erfindungsgemäßen Zusammensetzung sind insbesondere wie folgt verteilt: Komponente (A): ca. 5-80 Gew.-%, vorzugsweise ca. 20-60 Gew.-%, vor allem ca. 30-50 Gew-%; Komponente (B): ca. 5-80 Gew.-%, vorzugsweise ca. 10-40 Gew.-%, vor allem ca. 15-25-Gew.%; Komponente (C): ca. 10-80 Gew.-%, vorzugsweise ca. 20-60 Gew.-%, vor allem ca. 30-40 Gew.-%.

Im allgemeinen wird die erfindungsgemäße Zusammensetzung durch einfaches Mischen der Komponenten (A), (B) und (C) und/oder durch die Herstellung der einzelnen Komponenten in einer sogenannten Ein-Topf-Reaktion hergestellt.

Ein weiterer Gegenstand der vorliegenden Erfindung ist daher ein Verfahren zur Herstellung der erfindungsgemäßen Zusammensetzung durch Mischung der Komponenten (A), (B) und (C) und/oder Herstellung der Komponenten (A), (B) und/oder (C) in einer Ein-Topf-Reaktion vorzugsweise mit den oben beschriebenen Anteilen und nach den oben beschriebenen Verfahren.

Die erfindungsgemäße Zusammensetzung eignet sich in vorteilhafter Weise zur Behandlung von gegerbtem Leder.

Ein anderer Gegenstand der vorliegenden Erfindung bezieht sich daher auf ein Verfahren bzw. die Verwendung der erfindungsgemäßen Zusammensetzung zur Behandlung von gegerbtem Leder, insbesondere zur Neutralisation von gegerbtem Leder, vor allem zur Behandlung bzw. Neutralisation von mit Mineralstoff-Verbindungen gegerbtem Leder. Vorzugsweise eignet sich die erfindungsgemäße Zusammensetzung zur Behandlung von mit Chrom-, Aluminium-, Zirkonium-, Titan- und/oder Eisen-Salzen, insbesondere mit Chrom(III)-Salzen, vor allem mit Chrom(III)-sulfat gegerbtem Leder ("Wetblue"), Ebenfalls ist eine Anwendung für sogenannte "Wetwhite-Leder" möglich, d. h. mit organisch-chemischen Gerbstoffen vorgegerbte Leder. Insbesondere sind hierzu mit Aldehyd, z. B. Glutaraldehyd oder Formaldehyd, gegerbte Leder zu verstehen. Auch mit synthetischen Gerbstoffen gegerbte oder mit Kombinationen aus synthetischen Gerbstoffen und Gerbstoffen pflanzlichen Ursprungs gegerbte Leder zählen hierzu (Döppert, S. et al. (1994), Das Leder, 12, 272).

Die vorliegende Erfindung ermöglicht es nun in überraschender Weise, die Brillanz und Farbtiefe der Leder zu verbessern, wobei der oben beschriebene Bleicheffekt bekannter Neutralisationsgerbstoffe nicht bzw. nicht im wesentlichen beobachtet wird. Die Leder zeigen im allgemeinen einen sehr runden, angenehmen Griff und sind hervorragend festnarbig. Zudem wird eine verbesserte Narbenfeinheit der Lederoberfläche erzielt.

Die nachfolgenden Beispiele sollen die Erfindung näher beschreiben, ohne sie darauf zu beschränken.

### Beispiele

### Beispiel 1

64,8 g Naphthalin (0,5 mol) werden bei 100-110°C während 1 h mit 187,2 g Oleum 24% (2 mol SO₃) versetzt, auf 150°C geheizt und 1 h gehalten. Nach Abkühlen auf 120°C wird mit 180 g Wasser versetzt und mit ca. 265 g NaOH 50% pH 9 eingestellt.

190 g Formaldehyd 30% (1,9 mol) und 69,8 g Harnstoff-Lösung 65,5% (0,75 mol) werden zugegeben und 2 h bei 80°C kondensiert. Dann werden 325 g NaHSO₃ 40% (1,25 mol) zugegeben und mit NaOH 50% pH 9 eingestellt. Es wird 3 h bei 100-103°C gerührt.

Anschließend werden 285 g NaOH 50% zugetropft und gleichzeitig 265 g eines technischen Gemischs von Bernsteinsäure (20-30%), Glutarsäure (40-50%) und Adipinsäure (20-30%) zugegeben.

Feststoff-Gehalt: 46%.

### Beispiel 2

184 g Toluol (2 mol) werden bei 90°C während 45 min mit 360 g H₂SO₄ 95% (3,5 mol) versetzt und dann während 1 h auf 140-145°C geheizt. Diese Temperatur wird 3 h gehalten. Anschließend wird auf 100°C abgekühlt, mit 250 g Wasser versetzt und mit NaOH 50% pH 9 eingestellt.

Dann werden 198 g NaHSO₃ 40 % (0,75 mol), 150 g Formaldehyd 30 % (1,5 mol) und 45 g Harnstofff (0,75 mol) zugegeben. Das Reaktionsgemisch wird auf 90°C geheizt und 4 h gehalten, dann auf 80°C abgekühlt und mit 240 g des in Beispiel 1 genannten Dicarbonsäuregemisches sowie parallel mit 250 g NaOH 50% versetzt. Anschließend werden 150 g Wasser und 25,5 g Natriumformiat zugegeben.

Feststoff-Gehalt: 48%

### Beispiel 3

212 g Xylol (Isomerengemisch, 2 mol) werden bei 90°C während 30 min mit 490 g H₂SO₄ 95% (4,75 mol) versetzt und dann während 20 min auf 140°C geheizt. Diese Temperatur wird 3 h gehalten. Anschließend wird auf 100°C abgekühlt und mit 250 g Wasser versetzt. Mit NaOH 50% wird pH 9 eingestellt, dann werden 45 g Harnstoff (0,75 mol) und 175 g Formaldehyd 30% (1,75 mol) zugegeben, auf 80°C geheizt und 2 h bei dieser Temperatur gerührt. Anschließend werden 264 g NaHSO₃ 40% (1 mol) zugegeben und 4 h bei 90°C nachgerührt. Dann wird mit 200 g Wasser, 294 g des in Beispiel 1 genannten Dicarbonsäuregemisches und mit 50 g Ameisensäure versetzt und mit ca. 500 g NaOH 50% pH 7 eingestellt.

Feststoff-Gehalt: 47%.

### Vergleichsbeispiel A

142,5 g Naphthalin (1,11 mol) werden bei 100-110°C während 30 min mit 331 g H₂SO₄ konz. (3,24 mol) versetzt, auf 140°C geheizt und 5 h bei dieser Temperatur gehalten. Anschließend wird auf 80°C abgekühlt, mit 400 g Wasser, 117 g des in Beispiel 1 genannten Dicarbonsäuregemisches und mit 180 g Natriumformiat versetzt. Anschließend wird mit ca. 500 g NaOH 50% pH 7 eingestellt.

Feststoff-Gehalt: 48%.

### Vergleichsbeispiel B

850 g einer 30%igen wässrigen Lösung eines handelsüblichen Napthalinsulfonsäure-Formaldehyd-Kondensates werden auf 50°C geheizt und mit 265 g des in Beispiel 1 genannten Dicarbonsäuregemisches versetzt. Das Gemisch wird mit ca. 280 g NaOH 50% auf pH 7 gestellt.

Feststoff-Gehalt: 47%.

### Ausführungsbeispiele: Oberleder-Herstellung

Üblicherweise hergestelltes "Wetblue" aus Rindhäuten wird auf 1,8 mm gefalzt und mit 200% (Angaben jeweils auf Falzgewicht) Wasser gewaschen. Danach wird in 100% wäßriger Flotte mit 3% einer Gerbstoff-Mischung analog Beispiel 1 (berechnet auf Feststoff-Gehalt) 90 min bei 30°C gewalkt. Der pH-Wert der Flotte liegt dann bei ca. 4.5. Die Flotte wird abgelassen und mit 200% Wasser gewaschen. In 100% Flotte wird mit 2% eines handelsüblichen Polymer-Gerbstoffes auf Acrylsäurebasis 20 min gewalkt und dann mit 6% Vegetabil-Gerbstoff versetzt. Nach 40 min wird die Flotte abgelassen, gewaschen und mit handelsüblichen Farbstoffen und Fettungsmittel gefärbt und gefettet.

Man erhält ein Leder mit hervorragender Fein- und Festnarbigkeit, sehr dunkler, brillanter und gleichmäßiger Färbung, bei der auch die durch den Vegetabil-Gerbstoff bewirkte Trübung der Färbung nicht mehr auftritt. Das Leder ist sehr voll und hat einen runden Griff. Ähnliche Ergebnisse erhält man, wenn als Neutralisationskomponente die Produkte aus Beispiel 2 und 3 eingesetzt werden.

Vergleichsbeispiel A ergibt sehr ungleichmäßige, trübe Färbungen und sehr harte, klapprige Leder.

Vergleichsbeispiel B ergibt sehr aufgehellte Leder, die wesentlich losnarbiger sind als die Leder, die mit den Produkten gemäß Beispiele 1-3 behandelt wurden.

## Patentansprüche

1. Zusammensetzung enthaltend
(A) 5 bis 80 Gew.-% ein oder mehrere aromatische Sulfonate, ausgewählt aus einem sulfonierten Benzol und seinen Derivaten, sulfonierten polycyclischen aromatischen Verbindungen und ihren Derivaten, sulfonierten Oligophenylen oder seinen Derivaten, oder Mischungen davon, hergestellt aus einem Moläquivalent der aromatischen Verbindung und 1 bis 5 Moläquivalenten SO₃, wobei anschließend das Produkt auf einen pH von 6 bis 10 eingestellt wird, als Komponente (A);
(B) 5 bis 80 Gew.-% ein oder mehrere Aldehydharze, hergestellt aus einem Moläquivalent einer stickstoffhaltigen Verbindung, 2-3 Moläquivalenten eines aliphatischen Aldehyds und gegebenenfalls 1-3 Moläquivalenten von einem oder mehreren Salzen der schwefligen Säure bei einem pH-Wert von ca. 7 bis 10, als Komponente (B); und
(C) 10 bis 80 Gew.-% einen oder mehrere Puffer,
hergestellt durch Mischen der Komponenten (A), (B) und (C) und/oder Herstellung der Komponenten (A), (B) und/oder (C) in einer Ein-Topf-Reaktion.

2. Zusammensetzung nach Anspruch 1, **dadurch gekennzeichnet, daß** das aromatische Sulfonat eine zwei- bis dreifach sulfonierte aromatische Verbindung ist.

3. Zusammensetzung nach Anspruch 1 oder 2, **dadurch gekennzeichnet, daß** das Aldehydharz durch gleichzeitige oder nachfolgende Kondensation eines oder mehrerer aliphatischer Aldehyde mit einer oder mehreren stickstoffhaltigen Verbindungen und einem oder mehreren Salzen der schwefligen Säure hergestellt ist.

4. Zusammensetzung nach einem der Ansprüche 1 bis 3, **dadurch gekennzeichnet, daß** der aliphatische Aldehyd Formaldehyd und/oder Acetaldehyd ist.

5. Zusammmensetzung nach einem der Ansprüche 1 bis 4, **dadurch gekennzeichnet, daß** die stickstoffhaltige Verbindung ausgewählt ist aus Harnstoff, Melamin, Dicyanamid oder Guanidin.

6. Zusammensetzung nach einem der Ansprüche 1 bis 5, **dadurch gekennzeichnet, daß** der Puffer gemäß (C) ein oder mehrere Salze von einer oder mehreren Säuren mit Pufferwirkung enthält.

7. Zusammensetzung nach Anspruch 6, **dadurch gekennzeichnet, daß** die genannte Säure eine organische Säure, insbesondere eine organische Mono- und/oder Dicarbonsäure ist.

8. Zusammensetzung nach Anspruch 7, **dadurch gekennzeichnet, daß** die genannte Säure ausgewählt ist aus einem Formiat, Acetat, Carbonat, Oxicarbonat, Bicarbonat, Citrat, Glutamat, Lactat, Adipinat, Glutarat, Succinat, Oxalat, Malonat, Tartrat, Phthalat, Fumarat, Maleinat.

9. Zusammensetzung nach Anspruch 6, **dadurch gekennzeichnet, daß** die genannte Säure eine anorganische Säuse, insbesondere schweflige Säure, Phosphorsäure, Kieselsäure, Borsäure und/oder salpetrige Säure ist.

10. Verfahren zur Herstellung der Zusammensetzung gemäß einem der Ansprüche 1 bis 9, **dadurch gekennzeichnet, daß** die Komponenten (A), (B) und (C) gemischt und/oder die Komponenten (A), (B) und/oder (C) in einer Ein-Topf-Reaktion hergestellt werden.

11. Zusammensetzung, herstellbar nach dem Verfahren gemäß Anspruch 10.

12. Verfahren zur Behandlung von gegerbtem Leder, **dadurch gekennzeichnet, daß** das gegerbte Leder mit einer Zusammensetzung gemäß einem der Ansprüche 1 bis 9 und 11 behandelt wird.

13. Verfahren nach Anspruch 12, **dadurch gekennzeichnet, daß** es sich bei dem gegerbten Leder um mit Mineralstoff-Verbindungen gegerbtes Leder handelt.

14. Verfahren nach Anspruch 13, **dadurch gekennzeichnet, daß** die Mineralstoff-Verbindungen ausgewählt sind aus Chrom-, Aluminium-, Zirkonium-, Titan- und/oder Eisen-Salzen, insbesondere Chrom(III)-Salzen.

15. Verwendung einer Zusammensetzung gemäß einem der Ansprüche 1 bis 9 und 11 zur Behandlung von gegerbtem Leder.

16. Verwendung nach Anspruch 15, **dadurch gekennzeichnet, daß** es sich bei dem gegerbten Leder um mit Mineralstoff-Verbindungen gegerbtes Leder handelt.

17. Verwendung nach Anspruch 16, **dadurch gekennzeichnet, daß** die Mineralstoff-Verbindungen ausgewählt sind aus Chrom-, Aluminium-, Zirkonium-, Titan- und/oder Eisen-Salzen, insbesondere Chrom(III)-Salzen.

## Claims

1. A composition containing
(A) from 5 to 80% by weight of one or more aromatic sulfonates selected from sulfonated benzene and its derivatives, sulfonated polycyclic aromatic compounds and their derivatives, sulfonated oligophenyls and their derivatives, or mixtures thereof, prepared from one molar equivalent of the aromatic compound and from 1 to 5 molar equivalents of SO₃, the product subsequently being brought to a pH of from 6 to 10, as component (A) ;
(B) from 5 to 80% by weight of one or more aldehyde resins prepared from one molar equivalent of a nitrogen-containing compound, 2-3 molar equivalents of an aliphatic aldehyde and, if required, 1-3 molar equivalents of one or more salts of sulfurous acid at a pH of from about 7 to 10, as component (B); and
(C) from 10 to 80% by weight of one or more buffers,
prepared by mixing the components (A), (B) and (C) and/or preparing the components (A), (B) and/or (C) in a one-pot reaction.

2. A composition as claimed in claim 1, wherein the aromatic sulfonate is a disulfonated or trisulfonated aromatic compound.

3. A composition as claimed in claim 1 or 2, wherein the aldehyde resin is prepared by simultaneous or subsequent condensation of one or more aliphatic aldehydes with one or more nitrogen-containing compounds and one or more salts of sulfurous acid.

4. A composition as claimed in any of claims 1 to 3, wherein the aliphatic aldehyde is formaldehyde and/or acetaldehyde.

5. A composition as claimed in any of claims 1 to 4, wherein the nitrogen-containing compound is selected from urea, melamine, dicyanamide and guanidine.

6. A composition as claimed in any of claims 1 to 5, wherein the buffer according to (C) contains one or more salts of one or more acids having a buffer effect.

7. A composition as claimed in claim 6, wherein said acid is an organic acid, in particular an organic mono- and/or dicarboxylic acid.

8. A composition as claimed in claim 7, wherein said acid is selected from a formate, acetate, carbonate, basic carbonate, bicarbonate, citrate, glutamate, lactate, adipate, glutarate, succinate, oxalate, malonate, tartrate, phthalate, fumarate and maleate.

9. A composition as claimed in claim 6, wherein said acid is an inorganic acid, in particular sulfurous acid, phosphoric acid, silicic acid, boric acid and/or nitrous acid.

10. A process for preparing a composition as claimed in any of claims 1 to 9, wherein components (A), (B) and (C) are mixed and/or the components (A), (B) and/or (C) are prepared in a one-pot reaction.

11. A composition which can be prepared by a process as claimed in claim 10.

12. A process for treating tanned leather, wherein the tanned leather is treated with a composition as claimed in any of claims 1 to 9 and 11.

13. A process as claimed in claim 12, wherein the tanned leather is leather tanned with mineral compounds.

14. A process as claimed in claim 13, wherein the mineral compounds are selected from chromium, aluminum, zirconium, titanium and/or iron salts, in particular chromium(III) salts.

15. The use of a composition as claimed in any of claims 1 to 9 and 11 for treating tanned leather.

16. The use as claimed in claim 15, wherein the tanned leather is leather tanned with mineral compounds.

17. The use as claimed in claim 16, wherein the mineral compounds are selected from chromium, aluminum, zirconium, titanium and/or iron salts, in particular chromium(III) salts.

## Revendications

1. Composition contenant
(A) en tant que composant (A), de 5 à 80 % en poids d'un ou plusieurs sulfonates aromatiques choisis parmi un benzène sulfoné et ses dérivés, les composés aromatiques polycycliques sulfonés et leurs dérivés, les oligophényles sulfonés ou leurs dérivés, ou leurs mélanges, préparés à partir d'un équivalent en moles du composé aromatique et de 1 à 5 équivalents en moles de SO₃, le produit étant ensuite ajusté à un pH de 6 à 10;
(B) en tant que composant (B), de 5 à 80 % en poids d'une ou plusieurs résines aldéhydiques, préparées à partir d'un équivalent en moles d'un composé azoté, de 2 à 3 équivalents en moles d'un aldéhyde aliphatique et éventuellement de 1 à 3 équivalents en moles d'un ou plusieurs sels de l'acide sulfureux, à un pH d'environ 7 à 10 ; et
(C) de 10 à 80 % en poids d'un ou plusieurs tampons,
préparée par mélange des composants (A), (B) et (C) et/ou préparation des composants (A), (B) et/ou (C), dans le cadre d'une réaction monotope.

2. Composition selon la revendication 1, **caractérisée en ce que** le sulfonate aromatique est un composé aromatique deux à trois fois sulfoné.

3. Composition selon la revendication 1 ou 2, **caractérisée en ce que** la résine aldéhydique est préparée par condensation simultanée ou ultérieure d'un ou plusieurs aldéhydes aliphatiques avec un ou plusieurs composés azotés et un ou plusieurs sels de l'acide sulfureux.

4. Composition selon l'une des revendications 1 à 3, **caractérisée en ce que** l'aldéhyde aliphatique est le formaldéhyde et/ou l'acétaldéhyde.

5. Composition selon l'une des revendications 1 à 4, **caractérisée en ce que** le composé azoté est choisi parmi l'urée, la mélamine, le dicyanamide ou la guanidine.

6. Composition selon l'une des revendications 1 à 5, **caractérisée en ce que** le tampon selon (C) contient un ou plusieurs sels d'un ou plusieurs acides à effet tampon.

7. Composition selon la revendication 6, **caractérisée en ce que** l'acide mentionné est un acide organique, en particulier un acide mono- et/ou dicarboxylique organique.

8. Composition selon la revendication 7, **caractérisée en ce que** l'acide mentionné est choisi parmi un formiate, un acétate, un carbonate, un oxycarbonate, un bicarbonate, un citrate, un glutamate, un lactate, un adipate, un glutarate, un succinate, un oxalate, un malonate, un tartrate, un phtalate, un fumarate, un maléate.

9. Composition selon la revendication 6, **caractérisée en ce que** l'acide mentionné est un acide inorganique, en particulier l'acide sulfureux, l'acide phosphorique, l'acide silicique, l'acide borique et/ou l'acide nitreux.

10. Procédé de préparation de la composition selon l'une des revendications 1 à 9, **caractérisé en ce que** les composants (A), (B) et (C) sont mélangés, et/ou que les composants (A), (B) et/ou (C) sont préparés dans le cadre d'une réaction monotope.

11. Composition pouvant être préparée par le procédé selon la revendication 10.

12. Procédé pour le traitement du cuir tanné, **caractérisé en ce que** le cuir tanné est traité par une composition selon l'une des revendications 1 à 9 et 11.

13. Procédé selon la revendication 12, **caractérisé en ce que**, pour ce qui concerne le cuir tanné, il s'agit d'un cuir tanné par des composés de substances minérales.

14. Procédé selon la revendication 13, **caractérisé en ce que** les composés d'une substance minérale sont choisis parmi les sels de chrome, d'aluminium, de zirconium, de titane et/ou de fer, en particulier parmi les sels de chrome(III).

15. Utilisation d'une composition selon l'une des revendications 1 à 9 et 11 pour le traitement du cuir tanné.

16. Utilisation selon la revendication 15, **caractérisée en ce que**, pour ce qui concerne le cuir tanné, il s'agit d'un cuir tanné par des composés d'une substance minérale.

17. Utilisation selon la revendication 16, **caractérisée en ce que** les composés d'une substance minérale sont choisis parmi les sels de chrome, d'aluminium, de zirconium, de titane et/ou de fer, en particulier parmi les sels de chrome(III).
